# EUROPEAN PATENT SPECIFICATION

(11) **EP 0 894 500 B1**
(45) Date of publication and mention of the grant of the patent: **30.06.2004**
(21) Application number: 98202512.4
(22) Date of filing: 27.07.1998
(51) Int. Cl.: A61K 39/09

(54) **Streptococcus equi vaccine**
Streptococcus equi Impfstoff
Vaccin contre le Streptococcus equi

(30) Priority: 29.07.1997 EP 97202365
(43) Date of publication of application: 03.02.1999
(73) Proprietor: Akzo Nobel N.V., 6824 BM Arnhem (NL)
(72) Inventor: Jacobs, Antonius Arnoldus Christiaan, 5995 PS Kessel (NL)
(74) Representative: Mestrom, Joannes Jozef Louis

(56) References cited:
- EP-A- 0 786 518
- WO-A-87/00436
- US-A- 4 788 059
- CHALLACOMBE S J ET AL: "Salivary antibody responses in rhesus monkeys immunized with Streptococcus mutans by the oral, submucosal or subcutaneous routes." ARCHIVES OF ORAL BIOLOGY, (1979) 24 (12) 917-25. JOURNAL CODE: 83M. ISSN: 0003-9969., XP002084502 ENGLAND: United Kingdom

## Description

The present invention relates to the use of live attenuated *Streptococcus equi* for the manufacture of vaccines for submucosal application.

*Streptococcus equi* has been known for a long time to be the cause of an acute disease of the upper respiratory tract in horses (Sweeney et al., Compendium Equine 9: 689-693 (1987)) This highly contagious disease is characterised by fever, mucopurulent nasal discharge, lymphadenopathy and subsequent abscessation of the lymph nodes of the head and the neck (Sweeney et al., Compendium Equine 9: 845-851 (1987)).

The swelling of the lymph nodes is often so severe that the airways become obstructed. This phenomenon explains the common name of the disease; strangles.

The disease is only fatal in a minority of the cases, as described by Sigmund (Sigmund, O.H. and Fraser, C.M. eds.: The Merck Veterinary Manual, 5^{th} Ed. Merck and Company Inc., Rahway, N.J.: 313-315 (1979)). Contrary to this, morbidity is generally high, and can be as high as 100 % in susceptible populations.

Vaccines against the disease have also been known for a long time (Bazely, P.L.; Austr. Vet. J. 16: 243 (1940)) and (Bazely, P.L.; Austr. Vet. J. 18: 141-155 (1942).

Until recently, only two kinds of vaccines were available: a) vaccines based on classical bacterins and b) sub-unit vaccines based on the M-protein, an immunogenic protein.

Both kinds of vaccine have their own severe drawbacks. Bacterins are notorious for their adverse reactions and are known to provide relatively little protection (Subcommittee on the Efficacy of Strangles Bacterin, Report, American Association of Equine Practitioners).

Srivastava and Bamum (Can. J. Comp. Med. 45: 20-25 (1981)) demonstrated that commercial and autologous killed whole cell vaccines are not capable of inducing a sufficient level of antibodies to protect against strangles.

Woolcock Austr. Vet. J. 51: 554-559 (1975) showed that a vaccination scheme of three doses at ten-day intervals is essential to obtain at least partial protection. Boschwitz comes to the conclusion for the major immunogenic subunit of *S. equi,* the M-protein, that antibodies against this antigen are not sufficient to protect horses from natural or experimental infections (Cornell Vet. 81: 25-36 (1991)).

In a comparative study, in which both whole cell and M-subunit vaccines were compared, Srivastava and Barnum, Can. J. Comp. Med. 49: 351-356 (1981) again reported the very weak results with these kinds of vaccines: repeated booster vaccinations are necessary to obtain reasonable antibody titres and the longevity of the high titres is short. Further booster vaccinations should be given at least once a year (Sweeney et al., Compendium Equine 9: 845-851 (1987)). Moreover, there is hardly any correlation between antibody titers and protection. Even recent papers, in which killed *S. equi* was administered intraperitoneally or orally describe only partial protection against challenge with live *Streptococcus equi* (Wallace et al, Vet. Immunol. and Immunopath. 48: 139-154 (1995).

Classical vaccines based on bacterins or subunits are e.g. available trough Forth Dodge Laboratories, Coopers Animal Health and the Mobay Company (US Patent 4,944,942).

Mounting evidence in the literature indicates that immunity to *S. equi* is local, rather than systemic. The paramount importance of local immunity in conferring protection against streptococcal infection is i.a. well-documented by Bessen and Fischetti, in J. Exp. Medicine 167: 1945-1950 (1988), and in Infect. and Immun. 56: 2555-2672 (1988)).

It has been established that local immunity is mediated by mucosal antibodies as was summarised i.a. by Bernadette (J. Gen. Microbiol. 137:2125-2133 (1991).

And since the nasopharynx is the natural port of entrance for *Streptococcus equi,* it is now generally accepted that successful vaccination necessarily requires stimulation of the nasopharyngial immune response. (Galan et al., Inf. Immun. 47: 623-628 (1985), (Galan et al., Inf. Immun. 54: 202-206 (1986), (Timoney et al., In Kimura, y., Kotami, S., Shiokawa, y. (ed.). Recent advances in streptococci and streptococcal diseases. Reed Books, Danbury, N.H.)

The well-established importance of direct stimulation of the nasopharyngial immune response has contributed greatly to the current opinion, that only the intranasal application of a vaccine can provide at least partial protection.

A general problem with intranasally given live attenuated vaccines is the fact that the vaccine does not stay in the nose, but leaks away through sneezing, leaking and the like. This is an unwanted situation for two reasons: most of the vaccine virus becomes spread into the environment, and an overdose must be give to assure a sufficient level of vaccination. These disadvantages are however accepted in practice since as motivated above intranasal vaccination is currently considered the only way to obtain at least partial protection.

Challacombe, S.J., described in Archives of Oral Biology, vol. 24: 917-925 (1979) experiments with submucosal vaccination using adjuvanted killed *Streptococcus mutans* cells. This approach did not provide any protection against *Streptococcus mutans* infection.

EP0.786.518 describes a vaccine comprising live attenuated *Streptococcus equi* strain TW928. The vaccine described there is especially suitable for intranasal application. No mention is made of submucosal application.

Another live attenuated strain is described in WO/ 87/00436. In this Application it is once more stated that intranasal/oral vaccination with live *Streptococcus equi* is the only way to obtain possible protection, since other routes of vaccination and/or the use of subunit vaccines are known to be ineffective. The disadvantage of this vaccine however is, that it is a non-encapsulated mutant. Therefore, no immune response against the lipopolysaccharide moiety of the bacterium is obtained.

It is an objective of the present invention to circumvent the problems encountered with the above-mentioned live attenuated vaccines.

It was surprisingly found now, that in defiance of the current opinion about the importance of direct stimulation of the nasopharyngial immune response, a protection against strangles can be obtained if a vaccine based upon a live attenuated *Streptococcus equi* strain is administered submucosally instead of locally.

Thus, the present invention provides the use of a live attenuated bacterium of the species *Streptococcus equi* for the manufacture of a vaccine for submucosal application for combating *Streptococcus equi* infection.

When vaccination takes place in the mouth, the vaccine is advantageously administered submucosally into the cheek, or the upper or lower lip. Around the mouth, the lips of a horse are the easiest place to vaccinate from the point of animal handling. Therefore, in a more preferred form the invention relates to the use of a live attenuated bacterium of the species *Streptococcus equi* for the manufacture of a vaccine for labial application.

As a live attenuated *Streptococcus* strain, every strain can be used that possesses an attenuated character to the level that it does not cause unacceptable side-effects. The attenuated character may be obtained trough classical attenuation methods such as growth under different temperatures, growth in the presence of mutagenic agents, high energy radiation, UV-light and the like. The attenuated character may also be obtained by recombinant DNA techniques, e.g. the deletion of virulence factors.

During the manufacturing of the vaccine a pharmaceutically acceptable carrier can also be added. One possible carrier is a physiological salt-solution. Another pharmaceutically acceptable carrier is for instance the growth medium used for growing the bacterium.

If desired, an adjuvant and possibly one or more emulsifiers such as Tween^{(R)} and Span^{(R)} are also incorporated in the live vaccine. Suitable adjuvants are for example vitamin-E acetate solubilisate, aluminium hydroxide, -phosphate or -oxide, (mineral) oil emulsions such as Bayol^{(R)} and Marcol52^{(R)}, and saponins.

Thus in an even more preferred form of this embodiment, in addition to the live attenuated bacterium an adjuvant is used for the manufacture of said vaccine.

In a still even more preferred form of this embodiment, in addition to the live attenuated bacterium another pathogen or antigenic material from another pathogen is used for the manufacture of said vaccine. Such a pathogen may e.g. be another bacterium or a parasite.

Also it can be of viral origin. Usually, the other pathogen or antigenic material thereof will be a horse pathogen. A vaccine that comprises such an additional attenuated pathogen or antigenic material from another attenuated pathogen has the advantage that it induces protection against several infections at the same time.

In an even better form, the pathogens or antigenic material thereof is selected from the group of Potomac fever agent, *Rhodococcus equi, Clostridium tetanii, Mycobacterium pseudomallei*, *Streptococcus zoöepidemicus*, Vesicular Stomatitisvirus, Boma disease virus, Equine influenzavirus, African horse sickness virus, Equine arteritisvirus, Equine herpesvirus 1-4, Infectious anaemiavirus, Equine encephalomyelitisvirus and Japanese B encephalitisvirus.

In a most preferred form, the bacterium used for the manufacture of the vaccine according to the present invention is the *Streptococcus equi* strain TW 928 as disclosed in European Patent Application 0786518. This strain has been deposited under number CBS 813.95 with the Centraalbureau voor Schimmelcultures, P.O.box 273, 3740 AG Baarn, The Netherlands.

There are several ways to store live attenuated *Streptococcus equi.* Storage in a refrigerator is e.g. a well-known method. Also often used is storage at -70°C in a buffer containing glycerol. Bacteria can also be kept in liquid nitrogen. Freeze-drying is another way of conservation. Freeze-dried bacteria can be stored and kept viable for many years. Storage temperatures for freeze-dried bacteria may well be above zero degrees, without being detrimental to the viability. Freeze-drying can be done according to all well-known standard freeze-drying procedures. Optional beneficial additives, such as e.g. skimmed milk, trehalose, gelatin or bovine serum albumin can be added in the freeze-drying process.
Therefore, in a most preferred form, the vaccine, once manufactured, is in a freeze-dried form.

The doses that can be given depend partially on the level of attenuation. Generally spoken, doses ranging between 10³ and 10¹⁰ are suitable doses.

### EXAMPLES

### Comparative example

### Preparation of live attenuated vaccine:

Live attenuated *Streptococcus equi* strain TW 928 was grown under standard conditions in medium containing:

| | |
|---|---|
| tryptose | 5.0 g |
| Neutral soy peptone | 5.0 g |
| Yeast extract | 2.5 g |
| Beef extract | 5.0 g |
| D-glucose | 10.0 g |
| Ascorbic acid | 0.5 g |
| B-glycerolphosphate | 19.0 g |
| Magnesiumsulphate 7H2O | 0.25 g |
| Aqua dest. | 1 litre |

When used as a live attenuated strain, the bacteria were administered in the above mentioned medium. When given as an inactivated vaccine, the cells were first inactivated in 0.02% benzalkoniumchloride.

### Experimental design:

Twelve horses (13-16 months of age) with no history of strangles were divided into 4 groups of three horses each. At day 0, 21 and 42, horses of group I were vaccinated intranasally with 2x1ml live *S. equi* vaccine containing 10^{9.5} CFU/ml (1ml into each nostril). Horses of group II were vaccinated intramuscularly in the neck with 2 ml of the same live *S. equi* vaccine, and horses of group III were vaccinated intramuscularly in the neck with 2 ml of an inactivated whole cell vaccine containing 5x10⁹ CFU/ml in saponin adjuvant. The 3 horses of group IV were left untreated as unvaccinated challenge controls. At day 56 all 12 horses were challenged intranasally with 3 ml of a 10^{9.3} CFU/ml culture of *S. equi* strain Amica.

Rectal temperatures were determined at least twice weekly before challenge and daily after challenge. During the whole experiment the horses were daily observed for any abnormalities by a biotechnician. After each vaccination the horses were daily observed for local or systemic reactions until any reactions had disappeared for two subsequent days. Clinically examination by a veterinarian was done regularly before challenge and daily after challenge.

At 1 to 4 weeks after challenge (depending on clinical signs and or condition) the horses were killed and subjected to complete post-mortem investigation with special attention to signs of strangles and samples were taken for reisolation of *S. equi.*

### Clinical signs:

One month before first vaccination the horses were housed together to exchange microbiological flora. At day of first vaccination the horses had either no clinical signs or slight upper respiratory signs (e.g. nasal discharge) but further were in a good condition. After the vaccinations (until challenge) the horses had either no clinical signs or the same upper respiratory tract signs as before vaccination.

After challenge all 3 control horses, all 3 intranasally live vaccinated horses as well as all 3 horses that were vaccinated intramuscularly with the inactivated vaccine, developed clear signs of strangles characterised by sudden high temperatures, swollen lymph nodes of the head and the neck and purulent nasal discharge (where the last group appeared slightly less affected compared to the controls). In sharp contrast, the intramuscularly live vaccinated horses remained completely free from signs of strangles. The only signs observed were occasionally mild upper respiratory tract signs (i.e. same signs as observed before challenge and/or vaccination). The clinical scoring system is described in table 1. The clinical scores are summarised in Table 2. The total clinical score after challenge is obtained by addition of the clinical scores of each observation day after challenge, for each of the survived days.

Result: as is clear from table 2, intramuscular vaccination with a live attenuated *Streptococcus equi* vaccine reduces the clinical effects of a virulent *Streptococcus equi* challenge infection with 98%, whereas intranasal vaccination with the same vaccine only reduces the clinical effects with only 26%. Vaccination with inactivated cells of the same vaccine strain reduces the effects of challenge with 39%.

Intramuscular vaccination with a live attenuated *Streptococcus equi* vaccine gives contrary to intranasal vaccination or vaccination with inactivated vaccine, a virtually complete protection against infection with virulent *Streptococcus equi* strains.

### β-haemolytic streptococci in nasal washes:

The first week after challenge S. equi was isolated in low numbers from only a few horses.

Thereafter, the frequency and numbers strongly increased except for the intramuscularly live vaccinated horses and seemed to correlate with the presence of purulent nasal discharge, possibly originating from ruptured lymph node abscesses. The identity of these isolates was confirmed to be the challenge strain by using PCR.

**Result:** intramuscular application of live attenuated *Streptococcus equi* vaccines prevents spilling of vaccine into the environment and provides better dosage possibilities.

### Post-mortem examination, bacteriology and histology

The post-mortem findings confirmed the clinical findings: i.e. all 3 intramuscular live vaccinated horses appeared completely free from macroscopic or microscopic signs of strangles and except for the vaccine injection site, S. equi was not reisolated, whereas all other horses (except for horse 35) showed one or more (strongly) enlarged and abscessed lymph nodes from which S. equi was isolated. Microscopic examination of the retropharyngeal lymph nodes of horse 35 showed that this horse also had a (mild) lymphadenitis from which S. equi also was isolated
**Result:** the post-mortem results are fully in line with the clinical findings after challenge.

### Example 1:

### Submucosal labial vaccination of horses with a live attenuated S. equi vaccine

In this experiment the safety and efficacy of a live S. *equi* strain vaccine administered submucosally in the lip, was tested.

### Preparation of a vaccine S. equi strain for submucosal labial administration.

The content of vials of freeze dried vaccine were dissolved in 1.0 ml aqua dest. One dose = 200 µl of reconstituted vaccine.

For priming vaccination the titre of the reconstituted vaccine was 3.0x10⁹ CFU/ml and for booster vaccination 1.2x10⁹ CFU/ml.

### Test system

### Animals:

Ten Shetland type horses, seronegative for *S. equi,* 9-11 months old of age, with no history of strangles, were used for the experiment.

### EXPERIMENTAL SET-UP:

### Vaccination/challenge

After a two-week acclimatisation period, 5 horses were vaccinated submucosally in the lip (2 spots upper lip and 2 spots lower lip; 200 µl per spot) with the live attenuated vaccine strain. Five other horses were not vaccinated with the vaccine strain. At 4 weeks after priming vaccination the 5 vaccinates were boosted as described above. At 2 weeks after booster vaccination, all horses were challenged intranasally with *S. equi* strain Amica.

After vaccination the horses were daily observed for any systemic or local reactions. At the end of the experiment or earlier in case of severe clinical signs, the horses were killed and subjected to post-mortem investigation.

Two weeks after the booster vaccinations, all horses were challenged intranasally: 2 ml of a fresh 6 hours culture of wild-type *S. equi* strain Arnica in M17 medium + glucose into each nostril. Viable count was determined immediately after challenge by plate counting. The viable count was 7.7x10⁸ CFU/ml.

### Local and systemic reactions

At day of vaccination (just before), 6 hours after and then regularly during the experiment, the horses were observed for systemic and local reactions.

### Rectal temperatures

At day -1, 0 (just before and 6 hours after vaccination), 1 and 2 after each vaccination and regularly after challenge (until post-mortem), rectal temperatures were measured by a biotechnician.

### RESULTS

### Vaccination reactions

At 6 hours after vaccination with the live attenuated vaccine strain an increase in rectal temperature was found. Mean temperatures increased ±1°C compared to the controls at 6 hours after vaccination and ±1.7°C compared to pre-vaccination temperatures. Temperatures were normal again the next day.

After vaccination the horses appeared in a good condition and had a normal appetite and besides the effect on rectal temperature at 6 hours after vaccination no further systemic reactions were observed.

After both vaccinations with the vaccine strain small transient local reactions were found. Most reactions had disappeared at 3 weeks after priming vaccination and at 2 weeks after booster vaccination.

### Post-mortem examination and bacteriology

When post-mortem examinations were done, it turned out that the vaccinated horses had no strangles and thus were protected. All other horses had strangles, i.e. abscesses in the retropharyngeal lymph nodes from which *S. equi* was reisolated or fibrotic retropharyngeal lymph nodes indicative for a more chronic stage of strangles, where the abscesses already were (partly) resolved.

No residues or local tissues reactions were found at the sites of vaccination.

### DISCUSSION

From the results it is evident that an live attenuated *Streptococcus equi* vaccine induced complete protection against strangles (5/5 horses completely protected) whereas all other horses had strangles.

After vaccination with the attenuated strain, the horses appeared to be in a good condition and had a normal appetite and besides a rise in rectal temperature at 6 hours after vaccination no further systemic reactions were observed. Furthermore, after the vaccinations only small transient local reactions were found which resolved completely (no residues or local tissue damage/reactions were found at post-mortem).

### CONCLUSION

Submucosal vaccination in the lip with an attenuated live vaccine strain induced protection against strangles and is acceptable with regard to safety.

**Table 1.**

| Clinical scoring system. | | | |
|---|---|---|---|
| General impression | 0 = normal | Respiration type | 0 = costo-abdominal |
| | 1 = less active | | 2 = slightly abdominal |
| | 2 = slightly depressed | | 4 = abdominal |
| | 4 = depressed | | 6 = strongly abdominal |
| | 6 = severely depressed | | |
| | | Stridor | 0 = absent |
| Anorexia | 0 = normal | | 2 = slight |
| | 4 = loss of appetite | | 4 = moderate |
| | | | 6 = severe |
| Temperature | 0 = ≤ 38 5 | | |
| | 1 = 38 6 - 39 0 | | |
| | 2 = 39 1 - 39 5 | Auscultation | 0 = normal |
| | 3 = 39 6 - 40.0 | | 2 = slight rales |
| | 4 = 40 1 - 40 5 | | 4 = dry or wet rales |
| | 5 = 40 6 - 41 0 | | 6 = area with no sound |
| | 6 = > 41 0 | | |
| | | Lymph palpation | 0 = normal |
| Nasal discharge | 0 = absent | | 2 = stightly enlarged |
| | 1 = serous | | 4 = moderately enlarged |
| | 2 = mucopurulent | | 6 = strongly enlarged or ruptured |
| | 3 = purulent | | (if also painful, 1 point extra) |
| | (if excessive 1 point extra) | | |
| | | Palpation larynx | 0 = no coughing |
| Ocular discharge | 0 = absent | | 1 = one or two coughs |
| | 1 = serous | | 2 = several coughs |
| | 2 = mucopurulent | | (if painful, 1 point extra) |
| | 3 = purulent | | |
| | (if excessive 1 point extra) | Palpation trachea | 0 = no coughing |
| | | | 1 = one or two coughs |
| Throat swelling | 0 = absent | | 2 = several coughs |
| | 2 = slight | | (if painful, 1 point extra) |
| | 4 = moderate | | |
| | 6 = severe | Spontaneous cough | 0 = absent |
| | | | 2 = after inspection |
| Respiration rate (min.) | 0 = ≤ 35 | | 4 = before inspection |
| | 1 = 36-45 | | |
| | 2 = 46-55 | Lamness | 0 = absent |
| | 3 = 56-65 | | 3 = detectable at walk |
| | 4 = > 65 | | 6 = drags or jumps to carry lame legg |
| | | | (for each swollen joint. independent of tameness 3 points extra) |

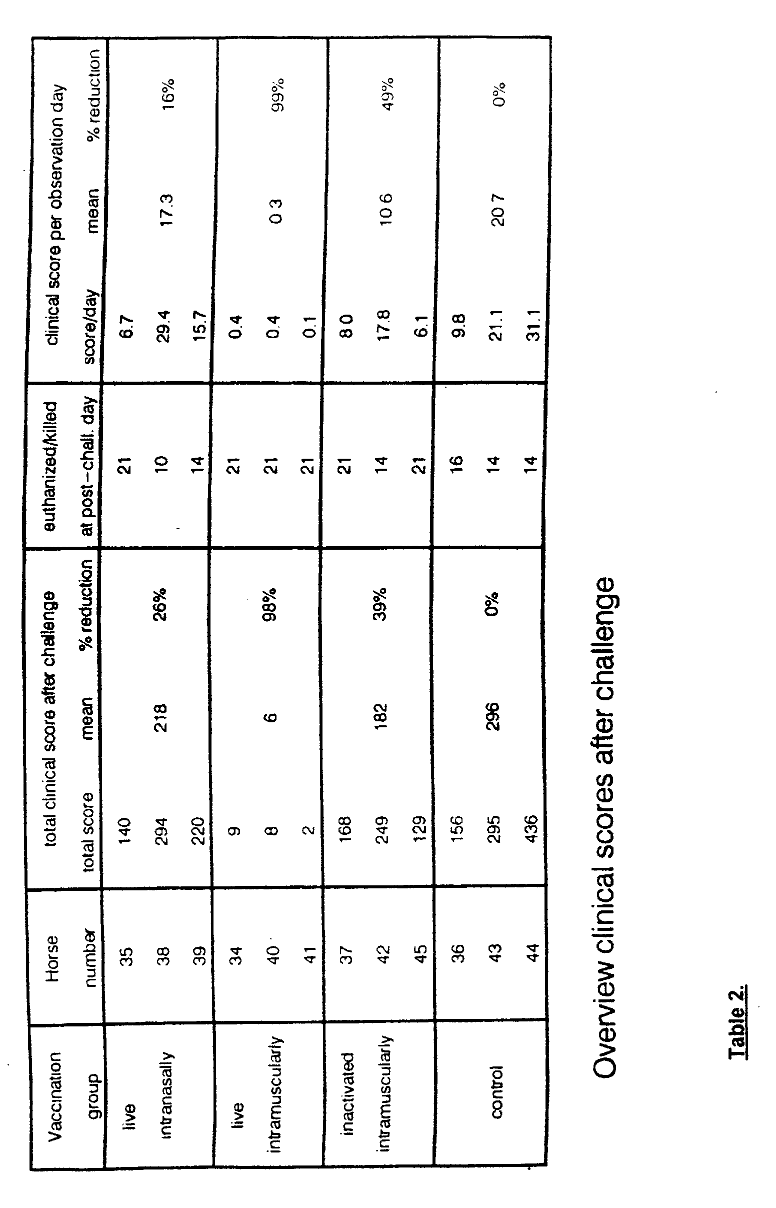

## Claims

1. Use of a live attenuated bacterium of the species *Streptococcus equi* for the manufacture of a vaccine for submucosal application for combating *Streptococcus equi* infection.

2. Use according to claim 1, **characterised in that** said bacterium is used for the manufacture of a vaccine for labial application.

3. Use according to claim 1 or 2, **characterised in that** in addition to the live attenuated bacterium an adjuvant is used for the manufacture of said vaccine.

4. Use according to claim 1-3, **characterised in that** in addition to the live attenuated bacterium another attenuated pathogen or antigenic material from another pathogen is used for the manufacture of said vaccine

5. Use according to claim 4, **characterised in that** said another attenuated pathogen or antigenic material from said another pathogen is selected from the group of Potomac fever agent, *Rhodococcus equi, Clostridium tetanii, Mycobacterium pseudomallei*, *Streptococcus zoöepidemicus*, Vesicular Stomatitisvirus, Borna disease virus, Equine influenzavirus, African horse sickness virus, Equine arteritisvirus, Equine herpesvirus 1-4, Infectious anaemiavirus, Equine encephalomyelitisvirus and Japanese B encephalitisvirus.

6. Use according to claim 1-5, **characterised in that** in addition during manufacturing the vaccine is freeze-dried.

## Revendications

1. Utilisation d'une bactérie vivante atténuée de l'espèce *Streptococcus equi* pour la fabrication d'un vaccin pour application sous-muqueuse pour combattre une infection à *Streptococcus equi.*

2. Utilisation selon la revendication 1, **caractérisée en ce que** ladite bactérie est utilisée pour la fabrication d'un vaccin pour application labiale.

3. Utilisation selon la revendication 1 ou 2, **caractérisée en ce qu'**en plus de la bactérie vivante atténuée, un adjuvant est utilisé pour la fabrication dudit vaccin.

4. Utilisation selon la revendication 1-3, **caractérisée en ce que** en plus de la bactérie vivante atténuée, un autre matériau atténué pathogène ou antigènique issu d'un autre agent pathogène est utilisé pour la fabrication dudit vaccin.

5. Utilisation selon la revendication 4, **caractérisée en ce que** ledit autre matériau atténué pathogène ou antigènique issu dudit autre agent pathogène est choisi dans le groupe de l'agent de la fièvre équine du Potomac, du *Rhodococcus equi,* du *Clostridium tetanii*, du *Mycobacterium pseudomallei*, du *Streptococcus zooepidemicus,* du virus de la stomatite vésiculaire, du virus de la maladie de Borna, du virus de la grippe équine, du virus de la peste équine, du virus de l'artérite équine, du virus de l'herpès équin types 1-4, du virus de l'anémie infectieuse, du virus de l'encéphalomyélite équine et du virus de l'encéphalite japonaise B.

6. Utilisation selon la revendication 1-5, **caractérisée en ce que**, en outre, au cours de la fabrication le vaccin est séché par lyophilisation.

## Patentansprüche

1. Verwendung eines lebenden, attenuierten Bakteriums der Spezies *Streptococcus* equi zur Herstellung eines Impfstoffes für eine submuköse Anwendung zur Bekämpfung von *Streptococcus equi* Infektion.

2. Verwendung gemäss Anspruch 1, **dadurch gekennzeichnet, dass** das besagte Bakterium zur Herstellung eines Impfstoffes für eine labiale Anwendung verwendet wird.

3. Verwendung gemäss Anspruch 1 oder 2, **dadurch gekennzeichnet, dass** zusätzlich zu dem lebenden, attenuierten Bakterium ein Adjuvans zur Herstellung des besagten Impfstoffes verwendet wird.

4. Verwendung gemäss Ansprüchen 1-3, **dadurch gekennzeichnet, dass** zusätzlich zu dem lebenden, attenuierten Bakterium ein anderes attenuiertes Pathogen oder antigenes Material von einem anderen Pathogen zur Herstellung des besagten Impfstoffes verwendet wird.

5. Verwendung gemäss Anspruch 4, **dadurch gekennzeichnet, dass** das besagte andere attenuierte Pathogen oder antigene Material von besagtem anderem Pathogen ausgewählt ist aus der Gruppe des Potomac Fieber Agens, *Rhodococcus equi, Clostridium tetanii, Mycobacterium pseudomallei, Streptococcus zooepidemicus,* Vesikulären Stomatitisvirus, Borna Disease Virus, Equinen Influenzavirus, Virus der afrikanischen Pferdekrankheit, Equinen Arteritisvirus, Equinen Herpesvirus 1-4, Infektiösen Anämievirus, Equinen Enzephalomyelitisvirus und Japanischen B Enzephalitisvirus.

6. Verwendung gemäss Ansprüchen 1-5, **dadurch gekennzeichnet, dass** zusätzlich der Impfstoff während der Herstellung gefriergetrocknet wird.
